# FASCICULE DE BREVET EUROPEEN

(11) **EP 1 366 744 B1**
(45) Date de publication et mention de la délivrance du brevet: **21.03.2007**
(21) Numéro de dépôt: 03291172.9
(22) Date de dépôt: 20.05.2003
(51) Int. Cl.: A61K 8/81, A61K 8/87, A61K 8/90, A61Q 5/06, A61Q 5/12

(54) **Composition capillaire aqueuse, épaissie par un copolymère linéaire séquencé amphiphile**
Wässrige Haarzusammensetzung verdickte mit einem linearen amphiphilen Blockcopolymer
Aqueous hair composition thickened with a linear amphiphilic block copolymer

(30) Priorité: 31.05.2002 FR 0206729
(43) Date de publication de la demande: 03.12.2003
(73) Titulaire: L'ORÉAL, 75008 Paris (FR)
(72) Inventeur: Dubief, Claude, 78510 Le Chesnay (FR); Giroud, Franck, 92110 Clichy (FR)
(74) Mandataire: Casalonga, Axel

(56) Documents cités:
- EP-A- 0 494 022
- EP-A- 0 761 199
- EP-A- 0 952 817
- WO-A-01/96429
- FR-A- 2 296 402
- FR-A- 2 709 954
- US-A- 3 907 984
- "APPLICAITON OF ACRYLATES/METHACRYLATES/BEHENETH-25 METHACRYLATE COPOLYMER AS A THICKENER IN COSMETIC FORMULATIONS" RESEARCH DISCLOSURE, KENNETH MASON PUBLICATIONS, HAMPSHIRE, GB, no. 420, avril 1999 (1999-04), page 494 XP000888570 ISSN: 0374-4353
- "Aqueous Gels of Block Copolymers:designing molecules and microstructures for tailored propwerties and performance"" ISIS 97, [Online] 1997, pages 56-57, Extrait de l'Internet: URL:http://www.isis.rl.ac.uk/ISIS> [extrait le 2001-07-19]

## Description

La présente invention concerne l'utilisation d'un copolymère linéaire séquencé comprenant au moins un bloc hydrophile et au moins un bloc hydrophobe, le ou les blocs hydrophiles représentant au moins 30 % en poids du copolymère linéaire séquencé, pour épaissir ou gélifier des compositions capillaires aqueuses, contenant au moins un polymère bénéfique pour la chevelure, et les compositions capillaires ainsi épaissies ou gélifiées.

De nombreux polymères épaississants ou gélifiants ont été proposés pour formuler des compositions aqueuses destinées au lavage, au soin et au coiffage des cheveux.

Parmi ces polymères, on utilise le plus souvent :
- les polymères naturels tels que les gommes de xanthane et de guar ou encore les dérivés cellulosiques, les amidons et les alginates. Ces composés altèrent souvent les propriétés cosmétiques des compositions épaissies. Ainsi, dans le domaine du traitement des cheveux, ils donnent souvent, malgré la présence d'agents conditionneurs, un toucher chargé et peu soyeux, parfois un peu sec, ainsi que, parfois, un aspect terne. Leur origine naturelle peut d'autre part induire des problèmes de reproductibilité entre les différents lots de matière première ce qui se traduit par une variabilité du pouvoir gélifiant, et
- les polymères synthétiques acryliques réticulés tels que les Carbopols^{®} commercialisés par la société Goodrich et les polymères d'acide 2-acrylamido-2-méthylpropane-sulfonique (AMPS) réticulés et au moins partiellement neutralisés, comme par exemple le produit commercialisé sous la dénomination Hostacerin^{®} AMPS par la société Clariant. Toutefois, ces agents gélifiants réticulés exigent, pour l'obtention de viscosités reproductibles, le respect d'un protocole spécifique et complexe de dispersion dans l'eau.

Différents agents gélifiants ont récemment été proposés afin de limiter ces problèmes de dispersions, comme par exemple les Carbopols^{®} ETD qui sont des Carbopols particuliers dits « faciles à disperser » (ETD *= Easy To Disperse*), ou encore les gélifiants réticulés dispersés dans une huile ou un mélange d'huiles, tels que le polyacrylamide commercialisé par la société Seppic sous la dénomination Sépigel^{®} 305.

Toutefois, la dispersion dans l'eau des Carbopols^{®} ETD nécessite de suivre un protocole spécifique de gonflement du polymère, tandis que les gélifiants fournis en dispersion dans une huile introduisent obligatoirement dans la composition des tensioactifs et une phase huileuse, cette dernière étant particulièrement indésirable dans le cas de compositions destinées à être appliquées sur les cheveux.

Le brevet EP0952817 décrit une composition cosmétique ou dermatologique à application topique, sous forme d'un gel aqueux, contenant au moins un copolymère amphiphile non-réticulé comportant à la fois des motifs hydrophiles et des motifs hydrophobes, au moins un agent tensioactif du type non ionique, et au moins un agent de conditionnement insoluble choisi parmi une silicone, un hydrocarbure, un alcool gras ou un ester gras. La composition sous forme de gel aqueux présente une excellente texture facilitant sa préhension et son application sur la peau ou les cheveux.

La demanderesse a constaté avec surprise qu'un groupe de copolymères séquencés particuliers, décrits plus en détail ci-après, permettent non seulement de surmonter les inconvénients des polymères épaississants et gélifiants de l'art antérieur décrits ci-dessus, mais ont en outre un effet bénéfique propre qui permet de renforcer l'action d'autres polymères présents dans les compositions cosmétiques capillaires.

La demanderesse a en outre observé que les copolymères séquencés, utilisés conformément à la présente invention pour l'épaississement ou la gélification de compositions capillaires - grâce à leur caractère amphiphile résultant de la présence, dans la molécule, d'une partie hydrophile et d'une partie hydrophobe - confèrent aux compositions épaissies une excellente homogénéité, qui est stable dans le temps et qui se traduit par un aspect plus transparent et plus esthétique des compositions.

La présente invention a par conséquent pour objet l'utilisation d'un copolymère linéaire séquencé, hydrosoluble ou finement hydrodispersible, comprenant au moins un bloc hydrophile et au moins un bloc hydrophobe, le ou les blocs hydrophiles représentant au moins 30 % en poids du copolymère linéaire séquencé, à l'exclusion des copolymères séquencés d'oxyde d'éthylène et d'oxyde de propylène, des copolymères séquencés à motifs uréthanne et des copolymères séquencés à motifs siloxane, pour l'épaississement ou la gélification de compositions capillaires aqueuses contenant au moins un polymère bénéfique pour la chevelure.

L'invention a également pour objet des compositions capillaires contenant, dans un milieu aqueux cosmétiquement acceptable,
- au moins un polymère fixant ou un polymère conditionneur; et
- au moins un copolymère linéaire séquencé, qui, introduit dans l'eau à 25 °C, à une concentration en poids égale à 0,1%, permet l'obtention d'une solution ou suspension macroscopiquement homogène et transparente ou translucide, c'est-à-dire ayant une valeur de transmittance de la lumière, à une longueur d'onde égale à 500 nm, à travers un échantillon de 1 cm d'épaisseur, d'au moins 70%, comprenant au moins un bloc hydrophile et au moins un bloc hydrophobe, le ou les blocs hydrophiles représentant au moins 30 % en poids du copolymère linéaire séquencé, à l'exclusion des copolymères séquencés d'oxyde d'éthylène et d'oxyde de propylène, des copolymères séquencés à motifs uréthanne et des copolymères séquencés à motifs siloxane, à raison de 0,01 à 10% en poids rapporté à la composition capillaire.

On entend par polymère possédant des propriétés fixantes, un polymère apportant du maintien à la chevelure, ou des propriétés conditionnantes. Par propriétés conditionnantes on entend une amélioration d'au moins l'une des propriétés suivantes : facilité de démêlage, douceur, brillance et caractère lisse des cheveux.

On entend par "quantité suffisante pour épaissir ou gélifier" ladite composition capillaire, une quantité permettant de conférer à la composition une viscosité dynamique, mesurée à une température de 25 °C au moyen d'un rhéomètre RHEOMAT RM 180, à une vitesse de cisaillement de 200 s⁻¹, supérieure à 0,1 Pa.s (1 poise) et de préférence supérieure à 0,2 Pa.s. (2 poises).

Les copolymères linéaires séquencés utilisables selon la présente invention pour épaissir ou gélifier des compositions capillaires aqueuses sont des copolymères dits "amphiphiles", à savoir des copolymères comportant à la fois des blocs hydrophobes et des blocs hydrophiles.

On entend par blocs hydrophobes selon la présente invention des blocs comprenant au moins 75 % en moles de monomères insolubles dans l'eau et par blocs hydrophiles des blocs comprenant au moins 75 % en moles de monomères hydrosolubles.

Les monomères hydrosolubles formant les blocs hydrophiles des copolymères séquencés utilisés dans la présente invention peuvent être de nature anionique, non-ionique ou cationique et peuvent être utilisés seuls ou sous forme de mélange contenant deux ou plusieurs monomères différents.

On peut citer à titre d'exemples de monomères hydrosolubles anioniques, les acides carboxyliques à insaturation éthylénique, tels que l'acide acrylique, l'acide méthacrylique, l'acide itaconique, l'acide fumarique, l'acide crotonique et l'acide maléique, l'acide 2-acrylamido-2-méthylpropanesulfonique, l'acide styrènesulfonique, l'acide vinylsulfonique et l'acide vinylphosphonique.

Les monomères hydrosolubles non-ioniques englobent, entre autres, l'acrylamide, les acrylamides N-alkylés en C₁₋₆ ou N,N-dialkylés en C₁₋₃, l'acrylate de polyéthylèneglycol, le méthacrylate de polyéthylèneglycol, le N-vinylacétamide, le N-méthyl-N-vinylacétamide, le N-vinylformamide, le N-méthyl-N-vinylformamide, les N-vinyllactames comportant un groupe cyclique de 4 à 9 atomes de carbone, l'alcool vinylique (copolymérisé sous forme d'acétate de vinyle puis hydrolysé), l'oxyde d'éthylène, l'acrylate d'hydroxyéthyle, l'acrylate d'hydroxypropyle, le méthacrylate d'hydroxyéthyle et le méthacrylate d'hydroxypropyle.

Enfin, les monomères hydrosolubles cationiques englobent par exemple le chlorure de diméthyldiallylammonium, le chlorure de méthylvinylimidazolium, la 2-vinylpyridine, la 4-vinylpyridine, la 2-méthyl-5-vinylpyridine, la vinylamine, les monomères de formule

H₂C=CR₁-CO-X₂

dans laquelle
R₁ représente un atome d'hydrogène ou un groupe méthyle,
X₂ représente un groupe hydrocarboné linéaire ou ramifié en C₁₋₆ portant au moins une fonction amine primaire, secondaire, tertiaire ou au moins un atome d'azote quaternaire, ou un groupe de formule NHR₂ ou de formule NR₂R₃ où R₂ et R₃ représentent indépendamment l'un de l'autre chacun un groupe hydrocarboné en C₁₋₆, linéaire ou ramifié, portant au moins une fonction amine primaire, secondaire, tertiaire ou au moins un atome d'azote quaternaire.

Les monomères insolubles dans l'eau formant les blocs hydrophobes des copolymères séquencés sont choisis de préférence parmi les monomères vinylaromatiques tels que le styrène et ses dérivés alkylés comme le 4-butylstyrène, l'α-méthylstyrène et le vinyltoluène, les diènes tels que le butadiène et le 1,3-hexadiène, et les dérivés alkylés des diènes tels que l'isoprène et le diméthylbutadiène, le chloroprène, les acrylates d'alkyle en C₁₋₁₀, d'aryle en C₆₋₁₀ ou d'aralkyle en C₆₋₁₀ et les méthacrylates d'alkyle en C_{1-10,} d'aryle en C₆₋₁₀ ou d'aralkyle en C₆₋₁₀, comme par exemple les (meth)acrylates de méthyle, d'éthyle, de n-butyle, de 2-éthylhexyle, de tert-butyle, d'isobornyle, de phényle ou de benzyle, l'acétate de vinyle, les vinyléthers de formule CH₂=CH-O-R et les allyléthers de formule CH₂=CH-CH₂-O-R où R représente un groupe alkyle en C₁₋₆, l'acrylonitrile, le chlorure de vinyle, le chlorure de vinylidène, la caprolactone, l'éthylène, le propylène, les monomères vinyliques fluorés ou à chaîne perfluorée, tels que les acrylates et méthacrylates de fluoroalkyle ou les α-fluoroacrylates d'alkyle.

Comme indiqué ci-dessus à propos de la définition des blocs hydrophobes et hydrophiles des copolymères séquencés, les monomères insolubles dans l'eau et les monomères hydrosolubles représentent au moins 75 % en moles respectivement des blocs hydrophobes et hydrophiles. Autrement dit, chaque bloc hydrophobe peut comprendre jusqu'à 25 % en moles d'un ou de plusieurs monomères hydrosolubles. Cette proportion est de préférence au plus égale 10 % en moles et idéalement inférieure ou égale à 5 % en moles.
De manière analogue, chaque bloc hydrophile peut comprendre jusqu'à 25 % en moles, de préférence jusqu'à 10 % en moles, et idéalement jusqu'à 5 % en moles, d'un ou de plusieurs monomères insolubles dans l'eau.
Les copolymères linéaires séquencés utilisés englobent bien entendu également ceux dans lesquels les blocs hydrophiles et les blocs hydrophobes sont constitués exclusivement respectivement de monomères hydrosolubles et de monomères insolubles dans l'eau. Ces blocs peuvent être des blocs homopolymères ou des blocs copolymères renfermant deux ou plus de deux monomères différents du même type.

On entend par monomère hydrosoluble un monomère qui, lorsqu'on l'introduit dans de l'eau à une température de 25 °C, et à une concentration en poids égale à 0,5 %, et éventuellement neutralisés, permet l'obtention d'une solution macroscopiquement homogène et transparente, c'est-à-dire ayant une valeur de transmittance de la lumière, à une longueur d'onde égale à 500 nm, à travers un échantillon de 1 cm d'épaisseur, d'au moins 70 %, de préférence d'au moins 80 %.

La masse moléculaire moyenne en nombre de chaque bloc, qu'il soit hydrophobe ou hydrophile, copolymère ou homopolymère, est de préférence comprise entre 500 et 100 000, en particulier entre 500 et 50 000, avec un indice de polydispersité (M_{w}/Mₙ) compris entre 1,01 et 3,0, de préférence entre 1,1 et 2,5.

Les copolymères linéaires séquencés utilisés dans la présente invention peuvent être
- des copolymères diblocs de formule AB,
- des copolymères triblocs de formule ABA ou BAB et
- des copolymères multiblocs comprenant, au moins deux blocs hydrophiles et au moins deux blocs hydrophobes disposés en alternance, chaque A représentant un bloc hydrophile et chaque B représentant un bloc hydrophobe, les blocs A d'un même polymère pouvant être identiques ou différents et les blocs B d'un même polymère pouvant être identiques ou différents.

On préfère en particulier des copolymères diblocs et des copolymères triblocs comportant un bloc central hydrophile et deux blocs latéraux hydrophobes.

Comme indiqué ci-avant, les copolymères séquencés amphiphiles utilisés pour l'épaississement de compositions capillaires aqueuses sont soit hydrosolubles soit finement hydrodispersibles.
Par "hydrosoluble" ou "finement hydrodispersible", on entend dans la présente demande, des polymères qui, introduits dans l'eau à 25°C, à une concentration en poids égale à 0,1%, permettent l'obtention d'une solution ou suspension macroscopiquement homogène et transparente ou translucide, c'est-à-dire ayant une valeur de transmittance de la lumière, à une longueur d'onde égale à 500 nm, à travers un échantillon de 1 cm d'épaisseur, d'au moins 70%, de préférence d'au moins 80%.

Cette aptitude à être dissous ou finement dispersé dans l'eau est liée à la proportion importante des séquences hydrophiles dans les copolymères séquencés amphiphiles.
Cette proportion doit être d'au moins 30 % en poids, et elle est de préférence supérieure ou égale à 60 % en poids, sans pour autant dépasser 97 % en poids.

Les polymères blocs de l'invention peuvent être préparés par les procédés de synthèse classiquement utilisés pour obtenir des polymères blocs. On peut citer par exemple les polymérisations anionique ou cationique, et la polymérisation radicalaire contrôlée (voir *"New Method of Polymer Synthesis",* Blackie Academic & Professional, Londres, 1995, volume 2, page 1, ou Trends Polym. Sci. 4, page 183 (1996) de C. J. Hawker), qui peut être mise en oeuvre suivant différents procédés comme par exemple la polymérisation radicalaire par transfert d'atomes (*Atom Transfert Radical Polymerization* ou ATRP) (voir *JACS,* 117, page 5614 (1995), de Matyjasezwski *et al.*), la méthode des radicaux tels que les nitroxydes (Georges et al., Macromolecules, 1993,26,2987).
On peut aussi utiliser ces procédés pour obtenir un seul des deux types de blocs du polymère de l'invention, l'autre bloc étant introduit dans le polymère final par l'intermédiaire de l'amorceur utilisé ou bien par réaction de couplage entre les blocs hydrophiles et hydrophobes.

La quantité de copolymères linéaires séquencés amphiphiles dans les compositions capillaires aqueuses de la présente invention dépend d'un grand nombre de paramètres parmi lesquels on peut citer la masse moléculaire des copolymères, le nombre et de la taille des blocs hydrophiles et hydrophobes, la quantité de polymères bénéfiques pour la chevelure, et surtout la viscosité de la composition que l'on souhaite obtenir.
On obtient généralement un épaississement ou une gélification satisfaisants avec une quantité de copolymères linéaires séquencés comprise entre 0,01 et 10 % en poids, de préférence entre 0,1 et 5 % en poids rapporté au poids de la composition capillaire.

Les compositions capillaires de la présente invention contiennent au moins un polymère bénéfique pour la chevelure.
Ce polymère peut être de nature cationique, anionique, non-ionique ou amphotère.
Les polymères cationiques sont choisis par exemple parmi ceux décrits dans les demandes de brevet EP 0 337 354, FR 2 270 846, FR 2 383 660, FR 2 598 611, FR 2 470 596 et FR 2 519 863.
Les polymères cationiques préférés sont choisis parmi ceux qui contiennent des motifs comportant des groupements amines primaires, secondaires, tertiaires et/ou quaternaires qui font partie de la chaîne macromoléculaire principale, ou bien sont portés par des groupes latéraux directement reliés à celle-ci.
Parmi les polymères cationiques, on peut citer plus particulièrement les polymères du type polyamine, polyaminoamide et polyammonium quaternaire. Il s'agit de produits connus.

Les polymères du type polyamine, polyaminoamide et polyammonium quaternaire, que l'on peut utiliser dans les compositions de la présente invention, sont ceux décrits dans les brevets FR 2 505 348 et FR 2 542 997. Parmi ces polymères, on peut citer en particulier :
(1) les homopolymères ou copolymères d'esters ou d'amides acryliques ou méthacryliques à fonction aminée, comportant des motifs de formules suivantes: 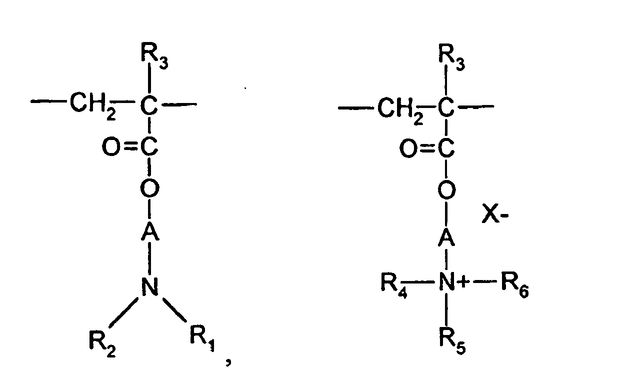 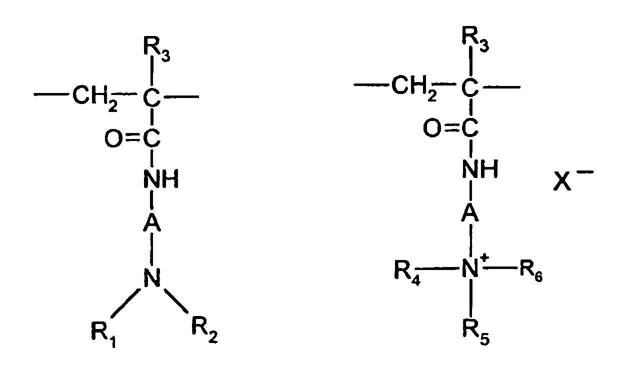 dans lesquelles:
   R₃, identiques ou différents, désignent un atome d'hydrogène ou un groupe CH₃ ;
   A, identiques ou différents, représentent un groupe alkyle, linéaire ou ramifié, comportant de 1 à 6 atomes de carbone, de préférence 2 ou 3 atomes de carbone, ou un groupe hydroxyalkyle comportant de 1 à 4 atomes de carbone ;
   R₄, R₅, R₆, identiques ou différents, représentent un groupe alkyle ayant de 1 à 18 atomes de carbone ou un groupe benzyle, et de préférence un groupe alkyle ayant de 1 à 6 atomes de carbone;
   R₁ et R₂, identiques ou différents, représentent un atome d'hydrogène
   ou un groupe alkyle ayant de 1 à 6 atomes de carbone, et de préférence un groupe méthyle ou éthyle;
X⁻ désigne un anion dérivé d'un acide minéral ou organique tel qu'un anion méthosulfate ou un halogénure comme le chlorure ou le bromure.
Les copolymères de la famille (1) peuvent contenir en outre un ou plusieurs motifs dérivant de comonomères choisis dans la famille des acrylamides, méthacrylamides, diacétone-acrylamides, acrylamides et méthacrylamides substitués sur l'atome d'azote par des groupes alkyle inférieur en C₁₋₄, des groupes dérivés des acides acryliques ou méthacryliques ou de leurs esters, de vinyllactames tels que la vinylpyrrolidone ou le vinylcaprolactame, d'esters vinyliques.
Parmi ces copolymères de la famille (1), on peut citer en particulier :
   - les copolymères d'acrylamide et de méthacrylate de diméthylaminoéthyle quaternisé au sulfate de diméthyle ou avec un halogénure de diméthyle, tels que celui vendu sous la dénomination HERCOFLOC^{®} par la société HERCULES,
   - les copolymères d'acrylamide et de chlorure de méthacryloyloxy-éthyltriméthylammonium décrits, par exemple, dans la demande de brevet EP-A-080976 et vendus sous la dénomination BINAQUAT^{®} P 100 par la société CIBA GEIGY,
   - le copolymère d'acrylamide et de méthosulfate de méthacryloyloxy-éthyltriméthylammonium vendu sous la dénomination RETEN^{®} par la société HERCULES,
   - les copolymères vinylpyrrolidone/acrylate ou méthacrylate de dialkylaminoalkyle quaternisés ou non, tels que les produits vendus sous la dénomination GAFQUAT^{®} par la société ISP comme, par exemple, GAFQUAT^{®} 734 ou GAFQUAT^{®} 755, ou bien les produits dénommés COPOLYMER 845, 958 et 937. Ces polymères sont décrits en détail dans les brevets FR 2 077 143 et FR 2 393 573,
   - les terpolymères méthacrylate de diméthylaminoéthyle/ vinylcaprolactame/vinylpyrrolidone tels que le produit vendu sous la dénomination GAFFIX^{®} VC 713 par la société ISP,
   - les copolymères vinylpyrrolidone/méthacrylamidopropyl-diméthylamine commercialisés notamment sous la dénomination STYLEZE^{®} CC 10 par ISP, et
   - les copolymères vinylpyrrolidone/méthacrylamide de diméthylaminopropyle quaternisé, tels que le produit vendu sous la dénomination GAFQUAT^{®} HS 100 par la société ISP.
(2) Les dérivés d'éthers de cellulose comportant des groupements ammonium quaternaires décrits dans le brevet FR 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammoniums quaternaires d'hydroxyéthylcellulose ayant réagi avec un époxyde substitué par un groupement triméthylammonium.
(3) Les dérivés cationiques de la cellulose tels que les copolymères de cellulose ou les dérivés de cellulose greffés avec un monomère hydrosoluble d'ammonium quaternaire, et décrits notamment dans le brevet US 4 131 576, tels que les hydroxyalkylcelluloses, comme les hydroxyméthyl-, hydroxyéthyl-ou hydroxypropylcelluloses greffées notamment avec un sel de méthacryloyléthyl-triméthylammonium, de méthacrylamidopropyl-triméthylammonium, de diméthyldiallylammonium.
   Les produits commercialisés répondant à cette définition sont plus particulièrement les produits vendus sous la dénomination Celquat^{®} L 200 et Celquat^{®} H 100 par la Société National Starch.
(4) Les polysaccharides cationiques décrits plus particulièrement dans les brevets US 3 589 578 et US 4 031 307, tels que les gommes de guar contenant des groupements cationiques trialkylammonium. On utilise, par exemple, des gommes de guar modifiées par un sel, par exemple le chlorure, de 2,3-époxypropyltriméthylammonium.
   De tels produits sont commercialisés notamment sous les dénominations commerciales de JAGUAR^{®} C 13 S, JAGUAR^{®} C 15, JAGUAR^{®} C 17 ou JAGUAR^{®} C162 par la société MEYHALL.
(5) Les polymères constitués de motifs pipérazinyle et de groupes divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets FR 2 162 025 et FR 2 280 361.
(6) Les polyaminoamides solubles dans l'eau, préparés en particulier par polycondensation d'un composé acide avec une polyamine. Ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épihalohydrine, d'un diépoxyde ou d'un dérivé bis-insaturé, l'agent réticulant étant utilisé dans des proportions allant de 0,025 à 0,35 mole par groupement amine du polyaminoamide. Ces polyaminoamides peuvent être alkylés ou, s'ils comportent une ou plusieurs fonctions amines tertiaires, quaternisées. De tels polymères sont notamment décrits dans les brevets FR 2 252 840 et FR 2 368 508.
(7) Les dérivés de polyaminoamides résultant de la condensation de polyalkylènes-polyamines avec des acides polycarboxyliques, suivie d'une alkylation par des agents bifonctionnels. On peut citer, par exemple, les polymères acide adipique/diakylaminohydroxyalkyl-dialkylènetriamine dans lesquels le groupe alkyle comporte de 1 à 4 atomes de carbone et désigne de préférence un groupe méthyle, éthyle ou propyle, et le groupe alkylène comporte de 1 à 4 atomes de carbone, et désigne de préférence le groupe éthylène. De tels polymères sont notamment décrits dans le brevet FR 1 583 363.
   Parmi ces dérivés, on peut citer plus particulièrement les polymères acide adipique/diméthylaminohydroxypropyl-diéthylène-triamine vendus sous la dénomination Cartaretine^{®} F, F4 ou F8 par la société Sandoz.
(8) Les polymères obtenus par réaction d'une polyalkylène-polyamine comportant deux groupements amine primaire et au moins un groupement amine secondaire, avec un acide dicarboxylique choisi parmi l'acide diglycolique et les acides dicarboxyliques aliphatiques saturés ayant de 3 à 8 atomes de carbone, le rapport molaire de la polyalkylène-polylamine à l'acide dicarboxylique étant compris entre 0,8:1 et 1,4:1. Le polyaminoamide résultant de cette réaction est ensuite amené à réagir avec l'épichlorhydrine dans un rapport molaire d'épichlorhydrine par rapport au groupement aminé secondaire du polyaminoamide compris entre 0,5:1 et 1,8:1. De tels polymères sont notamment décrits dans les brevets US 3 227 615 et US 2 961 347.
   Des polymères de ce type sont en particulier commercialisés sous la dénomination Hercosett^{®} 57 par la société Hercules Inc. ou bien sous la dénomination de PD 170 ou Delsette^{®} 101 par la société Hercules dans le cas du copolymère d'acide adipique/époxypropyl-diéthylène-triamine.
(9) Les cyclopolymères d'alkyldiallylamine ou de dialkyldiallylammonium tels que les homopolymères ou copolymères comportant, comme constituant principal de la chaîne, des motifs répondant aux formules (Va) ou (Vb): 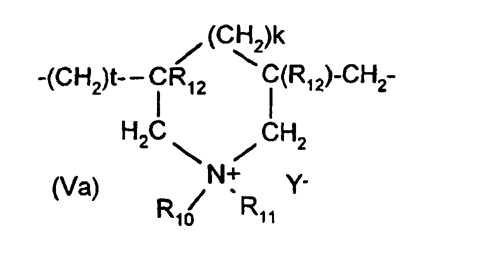 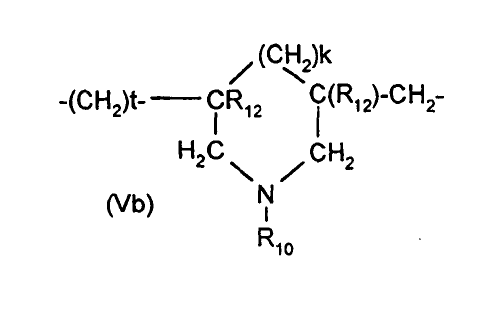 dans lesquelles
   k et t sont égaux à 0 ou 1, la somme k + t étant égale à 1 ;
   R₁₂ désigne un atome d'hydrogène ou un groupe méthyle ;
   R₁₀ et R₁₁, indépendamment l'un de l'autre, désignent un groupement alkyle ayant de 1 à 6 atomes de carbone, un groupement hydroxyalkyle en C₁₋₅, un groupement amidoalkyle inférieur en C₁-C₄, ou bien R₁₀ et R₁₁ peuvent désigner conjointement avec l'atome d'azote auquel ils sont rattachés, des groupements hétérocycliques, tels que pipéridinyle ou morpholinyle ;
   Y⁻ est un anion tel que bromure, chlorure, acétate, borate, citrate, tartrate, bisulfate, bisulfite, sulfate, phosphate.
   Ces polymères sont notamment décrits dans le brevet FR 2 080 759 et dans son certificat d'addition 2 190 406.
   Parmi les polymères définis ci-dessus, on peut citer plus particulièrement l'homopolymère de chlorure de diméthyldiallyl-ammonium vendu sous la dénomination MERQUAT^{®} 100 par la société CALGON (et ses homologues de faibles masses moléculaires moyenne en poids) et les copolymères de chlorure de diallyldiméthylammonium et d'acrylamide commercialisés sous la dénomination MERQUAT^{®} 550.
(10) Les polymères de diammonium quaternaire contenant des motifs récurrents répondant à la formule (VI) : 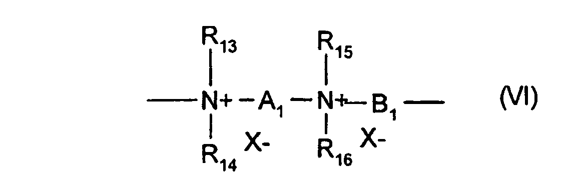 dans laquelle :
   R_{13,} R_{14,} R₁₅ et R_{16,} identiques ou différents, représentent des groupes aliphatiques, alicycliques ou arylaliphatiques contenant de 1 à 20 atomes de carbone ou des groupes hydroxyalkyle aliphatiques inférieurs, ou bien R_{13,} R_{14,} R₁₅ et R₁₆, ensemble ou séparément, forment avec les atomes d'azote auxquels ils sont rattachés des hétérocycles contenant éventuellement un second hétéroatome autre que l'azote, ou bien R_{13,} R₁₄, R₁₅ et R₁₆ représentent un groupe alkyle en C_{1-6,} linéaire ou ramifié, substitué par un groupement nitrile, ester, acyle, amide ou -CO-O-R₁₇-D ou -CO-NH-R₁₇-D où R₁₇ est un groupe alkylène et D un groupement ammonium quaternaire ;
   A₁ et B₁ représentent des groupements polyméthyléniques contenant de 2 à 20 atomes de carbone, pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A_{1,} R₁₃ et R₁₅ peuvent former avec les deux atomes d'azote auxquels ils sont rattachés un cycle pipérazinique ; en outre, si A₁ désigne un groupe alkylène
   ou hydroxyalkylène linéaire ou ramifié, saturé ou insaturé, B₁ peut également désigner un groupement :

   -(CH₂)ₙ-CO-D-OC-(CH₂)ₙ-

   dans lequel D désigne :
   a) un reste de glycol de formule -O-Z-O-, où Z désigne un groupe hydrocarboné linéaire ou ramifié, ou un groupement répondant à l'une des formules suivantes :

      -(CH₂-CH₂-O)ₓ-CH₂-CH₂-

      -[CH₂-CH(CH₃)-O]_{y}-CH₂-CH(CH₃)-

      où x et y désignent un nombre entier de 1 à 4, représentant un degré de polymérisation défini et unique ou un nombre quelconque de 1 à 4 représentant un degré de polymérisation moyen ;
   b) un reste de diamine bis-secondaire tel qu'un dérivé de pipérazine ;
   c) un reste de diamine bis-primaire de formule -NH-Y-NH-, où Y désigne un groupe hydrocarboné linéaire ou ramifié, ou bien le groupe divalent -CH₂-CH₂-S-S-CH₂-CH₂- ;
   d) un groupement uréylène de formule -NH-CO-NH- .
      De préférence, X⁻ est un anion tel que le chlorure ou le bromure.
      Ces polymères ont une masse moléculaire moyenne en nombre généralement comprise entre 1000 et 100000.

   Des polymères de ce type sont notamment décrits dans les brevets FR 2 320 330, FR 2 270 846, FR 2 316 271, FR 2 336 434 et FR 2 413 907 et les brevets US 2 273 780, US 2 375 853, US 2 388 614, US 2 454 547, US 3 206 462, US 2 261 002, US 2 271 378, US 3 874 870, US 4 001 432, US 3 929 990, US 3 966 904, US 4 005 193, US 4 025 617, US 4 025 627, US 4 025 653, US 4 026 945 et US 4 027 020.
   On peut utiliser plus particulièrement les polymères qui sont constitués de motifs récurrents répondant à la formule : 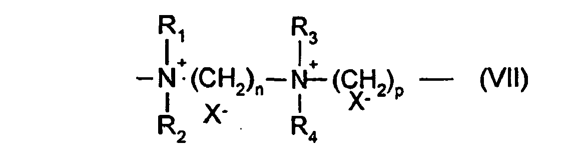 dans laquelle R₁, R₂, R₃ et R₄, identiques ou différents, désignent un groupe alkyle ou hydroxyalkyle ayant de 1 à 4 atomes de carbone environ, n et p sont des nombres entiers variant de 2 à 20 environ et, X⁻ est un anion dérivé d'un acide minéral ou organique.
   Un composé de formule (VII) particulièrement préféré est celui pour lequel R₁, R₂, R₃ et R₄ représentent un groupe méthyle et n=3, p=6 et X=Cl, dénommé chlorure d'hexadiméthrine (CTFA).
(11) Les polymères de polyammonium quaternaire, constitués de motifs de formule (VIII) : 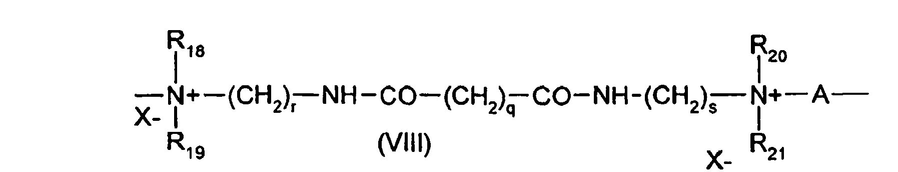 dans laquelle :
   R₁₈, R_{19,} R₂₀ et R_{21,} identiques ou différents, représentent un atome d'hydrogène ou un groupe méthyle, éthyle, propyle, β-hydroxyéthyle, β-hydroxypropyle ou -CH₂CH₂(OCH₂CH₂)ₚOH, où p est égal à 0 ou à un nombre entier compris entre 1 et 6, sous réserve que R₁₈, R₁₉, R₂₀ et R₂₁ ne représentent pas simultanément un atome d'hydrogène,
   r et s, identiques ou différents, sont des nombres entiers compris entre 1 et 6,
   q est égal à 0 ou à un nombre entier compris entre 1 et 34,
   X⁻ désigne un anion tel qu'un halogénure,
   A désigne un radical d'un dihalogénure ou représente de préférence -CH₂-CH₂-O-CH₂-CH₂-.
   De tels composés sont notamment décrits dans la demande de brevet EP-A-122 324.
      On peut citer parmi ceux-ci, par exemple, les produits Mirapol^{®} A 15, Mirapol^{®} AD1, Mirapol^{®} AZ1 et Mirapol^{®} 175 vendus par la société Miranol.
(12) Les polymères quaternaires de vinylpyrrolidone et de vinylimidazole tels que, par exemple, les produits commercialisés sous les dénominations Luviquat^{®} FC 905, FC 550 et FC 370 par la société B.A.S.F. On peut citer notamment les copolymères de vinylpyrrolidone et de chlorure de méthylvinylimidazolium.
(13) Les polyamines comme le Polyquart^{®} H vendu par HENKEL, référencées sous le nom de POLYETHYLENEGLYCOL (15) TALLOW POLYAMINE dans le dictionnaire CTFA.
(14) Les polymères réticulés ou non réticulés de sels de méthacryloyloxyalkyl(C₁₋₄) trialkyl(C₁₋₄)ammonium, tels que les polymères obtenus par homopolymérisation du méthacrylate de diméthylaminoéthyle quaternisé par le chlorure de méthyle, ou par copolymérisation de l'acrylamide et de méthacrylate de diméthylaminoéthyle quaternisé par le chlorure de méthyle, l'homopolymérisation ou la copolymérisation étant suivie d'une réticulation par un composé à insaturation oléfinique, en particulier le méthylène-bisacrylamide. On peut plus particulièrement utiliser un copolymère réticulé acrylamide/chlorure de méthacryloyloxy-éthyl-triméthylammonium (20/80 en poids) sous forme de dispersion contenant 50 % en poids dudit copolymère dans de l'huile minérale. Cette dispersion est commercialisée sous le nom de SALCARE^{®} SC 92 par la Société ALLIED COLLOIDS. On peut également utiliser un homopolymère réticulé du chlorure de méthacryloyloxyéthyl-triméthylammonium contenant environ 50 % en poids de l'homopolymère dans de l'huile minérale ou dans un ester liquide. Ces dispersions sont commercialisées sous les noms de SALCARE^{®} SC 95 et SALCARE^{®} SC 96 par la Société ALLIED COLLOIDS.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires, les dérivés cationiques de la chitine, et les silicones aminofonctionnelles.

Parmi tous les polymères cationiques susceptibles d'être utilisés dans les compositions cosmétiques de la présente invention, on préfère les dérivés d'éthers de cellulose comportant des groupements ammonium quaternaires, tels que les produits vendus sous la dénomination JR 400 par la Société UNION CARBIDE CORPORATION, les cyclopolymères cationiques, en particulier les homopolymères ou copolymères de chlorure de diméthyldiallylammonium, vendus sous les dénominations MERQUAT^{®} 100, MERQUAT^{®} 550 et MERQUAT^{®} S par la société CALGON, les polysaccharides cationiques tels que les gommes de guar modifiées par un sel de 2,3-époxypropyl-triméthylammonium, les copolymères quaternisés de vinylpyrrolidone et de vinylimidazole, les polycondensats de polyammonium quaternaire comportant de préférence les motifs récurrents de formules (VI) et (VIII) telles qu'indiquées ci-dessus, et leurs mélanges.

Les polymères bénéfiques anioniques généralement utilisés sont des polymères comportant des groupements dérivés d'acide carboxylique, d'acide sulfonique ou d'acide phosphorique et ont une masse moléculaire moyenne en nombre comprise entre environ 500 et 5 000 000.

Les groupements carboxyliques sont apportés par des monomères mono- ou diacides carboxyliques insaturés tels que ceux répondant à la formule : 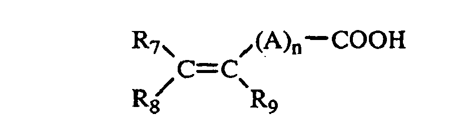 dans laquelle
n est un nombre entier de 0 à 10,
A₁ désigne un groupement méthylène, éventuellement relié à l'atome de carbone du groupement insaturé ou au groupement méthylène voisin lorsque n est supérieur à 1, par l'intermédiaire d'un hétéroatome tel que l'oxygène ou le soufre,
R₇ désigne un atome d'hydrogène, un groupement phényle ou benzyle,
R₈ désigne un atome d'hydrogène, un groupement alkyle inférieur ou carboxyle, et
R₉ désigne un atome d'hydrogène, un groupement alkyle inférieur, un groupement -CH₂-COOH, phényle ou benzyle.

Dans la formule précitée, un groupement alkyle inférieur désigne de préférence un groupement ayant 1 à 4 atomes de carbone et en particulier, les groupements méthyle et éthyle.

Les polymères bénéfiques anioniques à groupements carboxyliques préférés selon l'invention sont :
A) Les homo- ou copolymères d'acide acrylique ou méthacrylique ou leurs sels et en particulier les produits vendus sous les dénominations VERSICOL^{®} E ou K par la société ALLIED COLLOID et ULTRAHOLD^{®} par la société BASF, les copolymères d'acide acrylique et d'acrylamide vendus sous la forme de sel de sodium sous les dénominations RETEN^{®} 421, 423 ou 425 par la société HERCULES, et les sels de sodium des acides polyhydroxycarboxyliques.
B) Les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylèneglycol tel que le polyéthylèneglycol, et éventuellement réticulés. De tels polymères sont décrits en particulier dans le brevet français FR 1 222 944 et la demande de brevet allemand DE 2 330 956, les copolymères de ce type comportant dans leur chaîne un motif acrylamide éventuellement N-alkylé et/ou N-hydroxyalkylé tels que décrits notamment dans les demandes de brevets luxembourgeois n° 75370 et 75371 et proposés sous la dénomination QUADRAMER^{®} par la société AMERICAN CYANAMID. On peut également citer les copolymères d'acide acrylique et de méthacrylate d'alkyle en C₁-C₄ et les terpolymères de vinylpyrrolidone, d'acide acrylique et de méthacrylate d'alkyle en C₁-C_{20,} par exemple, de lauryle, tels que celui commercialisé par la société ISP sous la dénomination ACRYLIDONE^{®} LM et les terpolymères acide méthacrylique/acrylate d'éthyle/acrylate de tertiobutyle tels que le produit commercialisé sous la dénomination LUVIMER^{®} 100 P par la société BASF.
C) Les copolymères dérivés d'acide crotonique, tels que ceux comportant dans leur chaîne des motifs acétate ou propionate de vinyle, et éventuellement d'autres monomères tels que les esters allylique ou méthallylique, éther vinylique ou ester vinylique d'un acide carboxylique saturé, linéaire ou ramifié, à longue chaîne hydrocarbonée, comme ceux comportant au moins 5 atomes de carbone, ces polymères pouvant éventuellement être greffés et réticulés, ou encore un autre monomère ester vinylique, allylique ou méthallylique d'un acide carboxylique α- ou β-cyclique. De tels polymères sont décrits entre autres dans les brevets français FR 1 222 944, FR 1 580 545, FR 2 265 782, FR 2 265 781, FR 1 564 110 et FR 2 439 798. Des produits commerciaux entrant dans cette classe sont les résines 28-29-30, 26-13-14 et 28-13-10 commercialisée par la société NATIONAL STARCH.
D) Les copolymères dérivés d'acides ou d'anhydrides carboxyliques monoinsaturés en C₄-C₈ choisis parmi :
   - les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les brevets US n° 2,047,398, 2,723,248 et 2,102,113, et le brevet GB 839 805. Des produits commerciaux sont notamment ceux vendus sous les dénominations GANTREZ^{®} AN ou ES par la société ISP.
   - les copolymères comprenant (i) un ou plusieurs motifs anhydrides maléique, citraconique, itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne, les fonctions anhydrides de ces copolymères étant éventuellement monoestérifiées ou monoamidifiées.
      Ces polymères sont par exemple décrits dans les brevets français FR 2 350 384 et FR 2 357 241 de la demanderesse.
E) Les polyacrylamides comportant des groupements carboxylates.
   Les polymères comprenant les groupements sulfoniques sont des polymères comportant des motifs vinylsulfonique, styrène-sulfonique, naphtalène-sulfonique ou acrylamido-alkylsulfonique.
   Ces polymères peuvent être notamment choisis parmi :
   - les sels de l'acide polyvinylsulfonique ayant une masse moléculaire comprise entre environ 1 000 et 100 000, ainsi que les copolymères avec un comonomère insaturé tel que les acides acrylique ou méthacrylique et leurs esters, ainsi que l'acrylamide ou ses dérivés, les éthers vinyliques et la vinylpyrrolidone.
   - les sels de l'acide polystyrène-sulfonique tels que les sels de sodium vendus par exemple sous les dénominations FLEXAN^{®} 130 et FLEXAN^{®} 500 par la société NATIONAL STARCH. Ces composés sont décrits dans le brevet FR 2 198 719.
   - les sels d'acides polyacrylamide-sulfoniques tels que ceux mentionnés dans le brevet US 4,128,631, et plus particulièrement l'acide polyacrylamidoéthylpropanesulfonique vendu sous la dénomination COSMEDIA POLYMER^{®} HSP 1180 par la société HENKEL.
      Les polymères bénéfiques amphotères utilisables dans les compositions capillaires de la présente invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère comportant un ou plusieurs groupements carboxyliques ou sulfoniques. Les polymères bénéfiques amphotères peuvent également comporter des motifs zwitterioniques de type carboxybétaïne ou sulfobétaïne.
      Il peut également s'agir de polymères à chaîne principale cationique comportant des groupements aminé primaire, secondaire, tertiaire ou quaternaire, parmi lesquels au moins un porte, par l'intermédiaire d'un radical hydrocarboné, un groupement acide carboxylique ou acide sulfonique. Les polymères bénéfiques amphotères peuvent encore avoir une chaîne anionique dérivée d'acides carboxyliques α,β-insaturés dont l'un des groupements carboxyle a été amené à réagir avec une polyamine comportant un ou plusieurs groupements amine primaire.

Les polymères bénéfiques amphotères répondant à la définition donnée ci-dessus sont choisis notamment parmi les polymères suivants :
(1) les polymères résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloroacrylique, et d'un monomère basique dérivé d'un composé vinylique substitué contenant au moins un atome basique, tel que plus particulièrement les méthacrylate et acrylate de dialkylaminoalkyle, les dialkylaminoalkylméthacrylamide et acrylamide. De tels composés sont décrits dans le brevet américain US 3,836,537.
(2) les polymères comportant des motifs dérivant :
   (a) d'au moins un monomère choisi parmi les acrylamides ou les méthacrylamides substitués sur l'atome d'azote par un groupe alkyle,
   (b) d'au moins un comonomère acide contenant un ou plusieurs groupements carboxyliques réactifs, et
   (c) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou diéthyle.
      Les acrylamides ou méthacrylamides N-substitués sont notamment les composés dont les groupes alkyle comportent de 2 à 12 atomes de carbone, et plus particulièrement le N-éthylacrylamide, le N-tertiobutylacrylamide, le N-tertiooctylacrylamide, le N-octylacrylamide, le N-décylacrylamide, le N-dodécylacrylamide ainsi que les méthacrylamides correspondants.
      Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.
      Les comonomères basiques préférés sont des méthacrylates d'aminoéthyle, de butylaminoéthyle, de N,N'-diméthylaminoéthyle, de N-tertio-butylaminoéthyle.
      On utilise particulièrement les copolymères dont la dénomination CTFA (4^{ème} Ed., 1991) est Octylacrylamide/acrylates/butylaminoethyl-methacrylate copolymer, tels que les produits vendus sous la dénomination AMPHOMER^{®} ou LOVOCRYL^{®} 47 par la société NATIONAL STARCH.
(3) les polyaminoamides réticulés et alcoylés partiellement ou totalement dérivant de polyaminoamides de formule générale :

   -(CO-R₁₀-CO-Z-)-

   dans laquelle R₁₀ représente un groupe divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un groupe dérivant de l'addition de l'un quelconque desdits acides avec une amine bis-primaire ou bis-secondaire, et Z désigne un groupe dérivant d'une polyalkylène-polyamine bis-primaire, mono- ou bis-secondaire et de préférence représente :
   - dans les proportions de 60 à 100 % en moles, le groupe

      -NH-[(CH₂)ₓ-NH]ₚ-
   où x = 2 et p = 2 ou 3, ou bien x = 3 et p = 2
   ce groupe dérivant de la diéthylène-triamine, de la triéthylène-tétraamine ou de la dipropylène-triamine ;
   - dans les proportions de 0 à 40 % en moles, le groupe

      -NH-[(CH₂)ₓ-NH]ₚ-
   où x = 2 et p = 1 et qui dérive de l'éthylène-diamine, ou le groupe dérivant de la pipérazine : 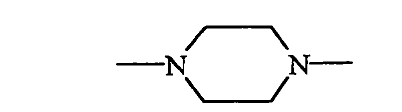
   - dans les proportions de 0 à 20 % en moles, le groupe

      -NH-(CH₂)₆-NH-

      dérivant de l'hexaméthylènediamine,
      ces polyaminoamides étant réticulés par réaction d'addition d'un agent réticulant bifonctionnel choisi parmi les épihalohydrines, les diépoxydes, les dianhydrides, les dérivés bis-insaturés, au moyen de 0,025 à 0,35 mole d'agent réticulant par groupement amine du polyaminoamide, et alcoylés par action d'acide acrylique, d'acide chloracétique ou d'une alcane-sultone ou de leurs sels.
      Les acides carboxyliques saturés sont choisis de préférence parmi les acides ayant 6 à 10 atomes de carbone tels que les acides adipique, triméthyl-2,2,4-adipique et triméthyl-2,4,4-adipique, téréphtalique, les acides à double liaison éthylénique comme, par exemple, les acides acrylique, méthacrylique, itaconique.
      Les alcane-sultones utilisées dans l'alcoylation sont de préférence la propane- ou la butane-sultone, les sels des agents d'alcoylation sont de préférence les sels de sodium ou de potassium.
(4) les polymères comportant des motifs zwittérioniques de formule : 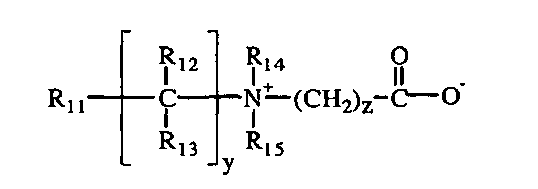 dans laquelle R₁₁ désigne un groupement insaturé polymérisable tel qu'un groupement acrylate, méthacrylate, acrylamide ou méthacrylamide, y et z représentent chacun un nombre entier de 1 à 3, R₁₂ et R₁₃ représentent un atome d'hydrogène, un groupe méthyle, éthyle ou propyle, R₁₄ et R₁₅ représentent un atome d'hydrogène ou un groupe alkyle de telle façon que la somme des atomes de carbone dans R₁₄ et R₁₅ ne dépasse pas 10.
   Les polymères comprenant de telles unités peuvent également comporter des motifs dérivés de monomères non zwittérioniques tels que l'acrylate ou le méthacrylate de diméthyl- ou diéthylaminoéthyle ou des acrylates ou méthacrylates d'alkyle, des acrylamides ou méthacrylamides, ou l'acétate de vinyle.
   A titre d'exemple, on peut citer les copolymères méthacrylate de méthyle/diméthylcarboxyméthylammonioéthylméthacrylate de méthyle, tels que le produit vendu sous la dénomination DIAFORMER^{®} Z301 par la société SANDOZ.
(5) les polymères dérivés du chitosane comportant des motifs monomères répondant aux formules suivantes : 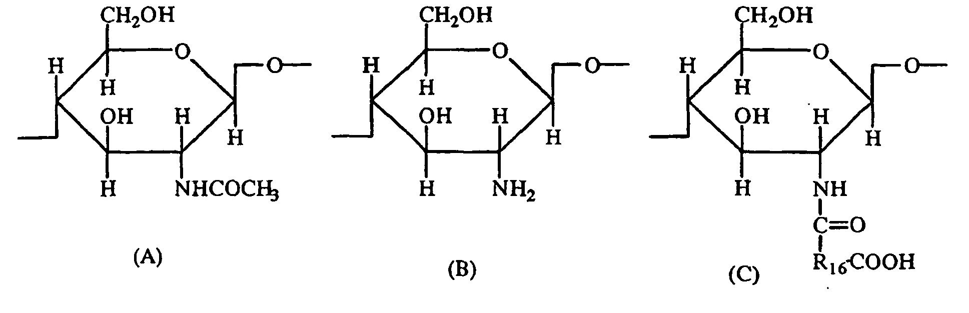 le motif (A) étant présent dans des proportions comprises entre 0 et 30%, le motif (B) dans des proportions comprises entre 5 et 50% et le motif (C) dans des proportions comprises entre 30 et 90%, étant entendu que dans ce motif (C), R₁₆ représente un groupe de formule : 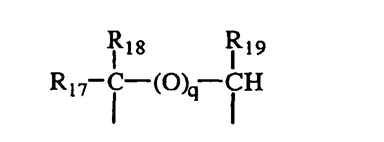 dans laquelle si q = 0, R_{17,} R₁₈ et R_{19,} identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des groupes R₁₇, R₁₈ et R₁₉ étant dans ce cas un atome d'hydrogène ;
   ou si q = 1,R₁₇, R₁₈ et R₁₉ représentent chacun un atome d'hydrogène, ainsi que les sels formés par ces composés avec des bases ou des acides.
(6) Les polymères obtenus par N-carboxylation du chitosane tels que le N-carboxyméthylchitosane ou le N-carboxybutylchitosane vendu sous la dénomination EVALSAN^{®} par la société JAN DEKKER.
(7) Les polymères décrits dans le brevet français FR 1 400 366 et répondant à la formule 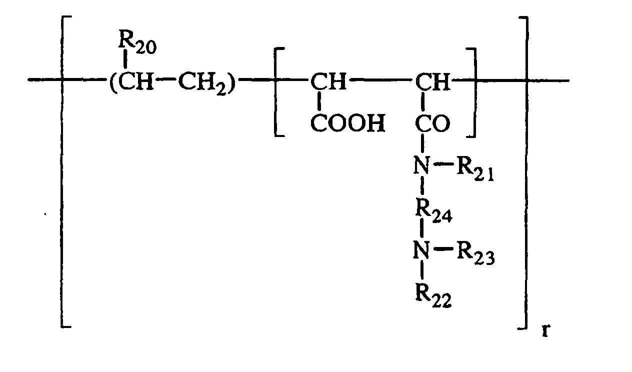 dans laquelle R₂₀ représente un atome d'hydrogène ou un groupe CH₃O, CH₃CH₂O ou phényle, R₂₁ désigne un atome d'hydrogène ou un groupe alkyle inférieur tel que méthyle et éthyle, R₂₂ désigne un atome d'hydrogène ou un groupe alkyle inférieur en C₁-C₆ tel que méthyle et éthyle, R₂₃ désigne un groupe alkyle inférieur en C₁-C₆ tel que méthyle, éthyle ou un groupe répondant à la formule : -R₂₄-N(R₂₂)₂, R₂₄ représentant un groupement
   - CH₂-CH₂-, -CH₂-CH₂-CH₂-, -CH₂-CH(CH₃)-, et R₂₂ ayant les significations mentionnées ci-dessus.
(8) Les polymères amphotères du type -D-X-D-X choisis parmi:
   (a) les polymères obtenus par action de l'acide chloroacétique ou le chloroacétate de sodium sur les composés comportant au moins un motif de formule :

      -D-X-D-X-D-

      où D désigne un groupe 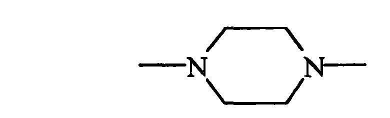 et X désigne le symbole E ou E', E ou E' identiques ou différents désignent un groupe bivalent qui est un groupe alkylène à chaîne droite ou ramifiée, comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques ; les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne.
   (b) Les polymères de formule :

      -D-X-D-X-
   où D désigne un groupe 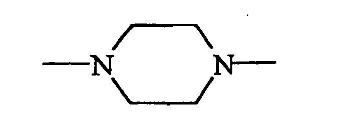 et X désigne le symbole E ou E' et au moins une fois E' ; E ayant la signification indiquée ci-dessus et E' est un groupe bivalent qui est un groupe alkylène à chaîne droite ou ramifiée, ayant jusqu'à 7 atomes de carbone dans la chaîne principale, substitué ou non par un ou plusieurs groupes hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloroacétate de soude.
(9) les copolymères alkyl(C₁-C₅)vinyléther/anhydride maléique modifiés partiellement par semi-amidification avec une N,N-dialkylaminoalkylamine telle que la N,N-diméthyaminopropylamine ou par semi-estérification avec une N,N-dialcanolamine. Ces copolymères peuvent également comporter d'autres comonomères vinyliques tels que le vinylcaprolactame.

Les polymères bénéfiques non-ioniques sont choisis, par exemple, parmi :
(a) la vinylpyrrolidone,
(b) les copolymères de vinylpyrrolidone et d'acétate de vinyle,
(c) les polyalkyloxazolines telles que les polyéthyloxazolines proposées par la société DOW CHEMICAL sous les dénominations PEOX^{®} 50 000, PEOX^{®} 200 000 et PEOX^{®} 500 000,
(d) les homopolymères d'acétate de vinyle tels que le produit proposé sous la dénomination APPRETAN^{®} EM par la société HOECHST
   ou le produit proposé sous le nom de RHODOPAS^{®} A 012 par la société RHONE POULENC,
(e) les copolymères d'acétate de vinyle et d'ester acrylique tels que le produit proposé sous le nom de RHODOPAS^{®} AD 310 de la société RHONE POULENC,
(f) les copolymères d'acétate de vinyle et d'éthylène tels que le produit proposé sous le nom de APPRETAN^{®} TV par la société HOECHST,
(g) les copolymères d'acétate de vinyle et d'ester maléique, par exemple, de maléate de dibutyle tels que le produit proposé sous le nom de APPRETAN^{®} MB EXTRA par la société HOECHST,
(h) les copolymères de polyéthylène et d'anhydride maléique,
(i) les poly(acrylate d'alkyle) et poly(méthacrylate d'alkyle) tels que le produit proposé sous la dénomination MICROPEARL^{®} RQ 750 par la société MATSUMOTO ou le produit proposé sous la dénomination LUHYDRAN^{®} A 848 S par la société BASF,
(j) les copolymères d'esters acryliques tels que, par exemple, les copolymères d'acrylates d'alkyle et de méthacrylates d'alkyle tels que les produits proposés par la société ROHM & HAAS sous les dénominations PRIMAL^{®} AC-261 K et EUDRAGIT^{®} NE 30 D, par la société BASF sous les dénominations ACRONAL^{®} 601, LUHYDRAN^{®} LR 8833 ou 8845, par la société HOECHST sous les dénominations APPRETAN^{®} N9212 et N9213 ;
(k) les copolymères d'acrylonitrile et d'un monomère non ionique choisis, par exemple, parmi le butadiène et les (méth)acrylates d'alkyle ; on peut citer les produits proposés sous les dénominations NIPOL^{®} LX 531 B par la société NIPPON ZEON ou ceux proposés sous la dénomination CJ 0601 B par la société ROHM & HAAS,
(l) les polyamides tels que le produit ESTAPOR^{®} LO 11 proposé par la société RHONE POULENC,
(m) les gommes de guar non ioniques chimiquement modifiées ou non modifiées. Les gommes de guar non modifiées sont par exemple les produits vendus sous la dénomination VIDOGUM^{®} GH 175 par la société UNIPECTINE et sous la dénomination JAGUAR^{®} C par la société MEYHALL. Les gommes de guar modifiées sont de préférence modifiées par des groupements hydroxyalkyle en C_{1-6,} de préférénce par des groupements hydroxyméthyle, hydroxyéthyle, hydroxypropyle et hydroxybutyle. De telles gommes de guar non ioniques éventuellement modifiées par des groupes hydroxyalkyle sont par exemple vendues sous les dénominations commerciales JAGUAR^{®} HP8, JAGUAR^{®} HP60, JAGUAR^{®} HP120, JAGUAR^{®} DC 293 et JAGUAR^{®} HP 105 par la société MEYHALL, ou sous la dénomination GALACTOSOL^{®} 4H4FD2 par la société AQUALON.

On peut également utiliser, en tant que polymères bénéfiques, des polymères filmogènes de type silicone greffée comprenant une partie polysiloxane et une partie constituée d'une chaîne organique non siliconée, l'une des deux parties constituant la chaîne principale du polymère et l'autre étant greffée sur ladite chaîne principale.

Ces polymères sont par exemple décrits dans les demandes de brevet EP-A-0 412 704, EP-A-0 412 707, EP-A-0 640 105 et WO 95/00578, EP-A-0582 152 et WO 93/23009 et les brevets US 4,693,935, US 4,728,571 et US 4,972,037.

Ces polymères peuvent être anioniques, cationiques, non-ioniques ou amphotères, mais sont de préférence anioniques ou non-ioniques.

De tels polymères sont par exemple les copolymères susceptibles d'être obtenus par polymérisation radicalaire à partir du mélange de monomères formé
a) de 50 à 90 % en poids d'acrylate de tertiobutyle,
b) de 0 à 40 % en poids d'acide acrylique,
c) de 5 à 40 % en poids d'un macromère siliconé de formule 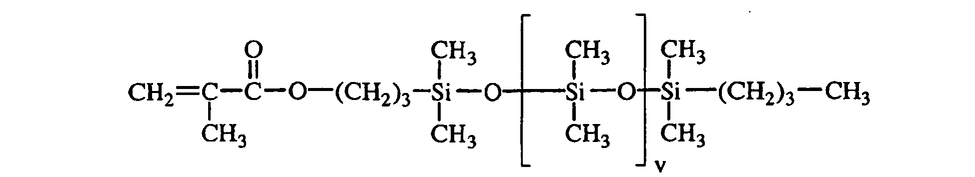
où v est un nombre allant de 5 à 700, les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

On peut également utiliser des polymères contenant des motifs uréthane. Ces polyuréthanes peuvent être fonctionnalisés ou non, siliconés ou non, cationiques, non-ioniques, anioniques ou amphotères. Les polyuréthanes particulièrement visés sont ceux décrits dans les demandes EP 0 751 162, EP 0 637 600, EP 0 648 485 et FR 2 743 297 dont la demanderesse est titulaire, ainsi que dans les demandes EP 0 656 021 et WO 94/03510 de la société BASF, et dans les demandes EP 0 619 111 de la société National Starch. Comme polyuréthanes convenant particulièrement bien à la présente invention, on peut citer les produits commercialisés sous les dénominations LUVISET PUR^{®} et LUVISET^{®} Si PUR par la société BASF.

Les polymères bénéfiques, c'est-à-dire fixants et/ou conditionneurs sont présents dans les compositions capillaires de la présente invention en une quantité suffisante pour obtenir l'effet cosmétique obtenu.
Cette quantité est généralement comprise entre 0,01 % et 20 % en poids, de préférence entre 0,1 et 10 % en poids.

Le milieu aqueux cosmétiquement acceptable peut être constitué uniquement d'eau ou d'un mélange d'eau et d'un ou de plusieurs solvants cosmétiquement acceptables miscibles avec l'eau tels que les alcools inférieurs en C₁-C₄, en particulier l'éthanol, l'isopropanol, le tertio-butanol et le n-butanol.

Les compositions selon l'invention peuvent également contenir des additifs cosmétiques et/ou des adjuvants de formulation tels que les silicones volatiles ou non volatiles, les agents tensioactifs anioniques, cationiques, amphotères ou non-ioniques, les agents nacrants, les agents opacifiants, les pigments et colorants, les huiles, les cires dont les céramides, les filtres UV organiques ou minéraux, les agents anti-radicalaires, les vitamines, les protéines, les agents anti-pelliculaires, les agents plastifiants, les agents d'ajustement et de fixation du pH, les agents anti-oxydants, les agents conservateurs, les précurseurs de colorants capillaires et les agents oxydants.
L'homme de métier veillera à choisir les éventuels additifs et leur quantité de manière à ce qu'ils ne nuisent pas aux propriétés intéressantes des compositions de la présente invention.

Les compositions capillaires de la présente invention, épaissies ou gélifiées grâce à l'utilisation de copolymères linéaires séquencés tels que décrits ci-dessus, peuvent se présenter sous n'importe quelle forme permettant une application aisée sur les cheveux. Il s'agit de préférence de lotions épaissies, de gels aqueux ou hydro-alcooliques, de crèmes ou de pâtes plus ou moins dures.

Ces compositions peuvent être conditionnées dans un dispositif aérosol en présence d'un ou de plusieurs agents propulseurs. Ces agents propulseurs sont de préférence choisis parmi le diméthyléther, les alcanes en C_{3-5,} le 1,1-difluoroéthane, les mélanges de diméthyléther et d'alcanes en C₃₋₅ et les mélanges de 1,1-difluoroéthane et de diméthyléther et/ou d'alcanes en C₃₋₅.

La présente invention est illustrée ci-après à l'aide d'un exemple.

### Exemple

On prépare les gels de coiffage suivants contenant, en tant que polymère fixant, 2 % en poids de polyvinypyrrolidone (PVP), gélifiés respectivement avec un polymère selon l'état de la technique (ex. comparatif), un copolymère séquencé diblocs selon l'invention (Composition A) et un copolymère séquencé triblocs selon l'invention (Composition B).

| | ex. comparatif | Composition A | Composition B |
|---|---|---|---|
| poly(acide acrylique) réticulé^{(a)} | 1 % | | |
| copolymère séquencé diblocs^{(b)} | | 1 % | |
| copolymère séquencé triblocs^{(c)} | | | 1 % |
| polyvinylpyrrolidone ^{(d)} (polymère fixant) | 2 % | 2 % | 2 % |
| parfum | q.s. | q.s. | q.s. |
| éthanol | 10 % | 10 % | 10 % |
| aminométhylpropanol | q.s.p. pH 7 | q.s.p. pH 7 | q.s.p. pH 7 |
| eau | q.s.p. 100 % | q.s.p. 100 % | q.s.p. 100 % |

| | | | |
|---|---|---|---|
| ^{(a)}SYNTHALEN^{®} K commercialisé par la société 3V ^{(b)}poly(styrène-b-acide acrylique), fourni par Polymer Source Inc. : masse moléculaire du bloc styrène : 1500 masse moléculaire du bloc acide acrylique : 44 000 ^{(c)}poly(styrène-b-acide acrylique-b-styrène), fourni par Polymer Source Inc. masse moléculaire de chaque bloc styrène : 1000 masse moléculaire du bloc acide acrylique : 40 000 ^{(d)}PVP K30 commercialisé par la société ISP | | | |

Chacune des compositions A et B ainsi que la composition de l'exemple comparatif sont appliquées sur 10 chevelures de cheveux européens châtains courts (5 à 10 cm) à raison de 5 g par chevelure.
Les propriétés de maintien des cheveux traités à l'aide des trois compositions ci-dessus sont évaluées par 5 experts selon une échelle de notation allant de 0 (absence de fixation) à 5 (fixation très élevée). Les chiffres indiqués ci-dessous correspondent à la moyenne des notes données par l'ensemble des experts.

| | |
|---|---|
| Composition A (selon l'invention) : | 4,3 |
| Composition B (selon l'invention) : | 4,7 |
| Exemple comparatif : | 3,4 |

Ces résultats montrent que les copolymères linéaires séquencés amphiphiles, utilisés conformément à l'invention pour épaissir des compositions coiffantes, renforcent l'effet recherché, à savoir l'effet fixant. En effet, le niveau de fixation obtenu avec les compositions selon la présente invention (polymère fixant + copolymère séquencés amphiphiles diblocs ou triblocs) est significativement plus élevé que celui obtenu avec une composition selon l'état de la technique (polymère fixant + polymère épaississant de type Carbopol).

L'utilisation de copolymères linéaires séquencés amphiphiles dans des compositions de coiffage permet ainsi d'obtenir un niveau de fixation plus élevé sans augmenter la concentration en polymère fixant. Cela présente toujours un avantage lorsque le polymère fixant donne lieu, au-delà d'une certaine quantité, à des effets indésirables tels qu'un aspect terne ou un effet de poudrage.

## Revendications

1. Composition capillaire contenant, dans un milieu aqueux cosmétiquement acceptable,
• au moins un polymère fixant ou un polymère conditionneur et
• au moins un copolymère linéaire séquencé, qui, introduit dans l'eau à 25 °C, à une concentration en poids égale à 0,1%, permet l'obtention d'une solution ou suspension macroscopiquement homogène et transparente ou translucide, c'est-à-dire ayant une valeur de transmittance de la lumière, à une longueur d'onde égale à 300 nm, à travers un échantillon de 1 cm d'épaisseur, d'au moins 70% comprenant au moins un bloc hydrophile et au moins un bloc hydrophobe, le ou les blocs hydrophiles représentant au moins 30 % en poids du copolymère linéaire séquencé, à l'exclusion des copolymères séquencés d'oxyde d'éthylène et d'oxyde de propylène, des copolymères séquencés à motifs uréthanne et des copolymères séquencés à motifs siloxane, à raison de 0,01 à 10 % en poids rapporté à la composition capillaire

2. Composition capillaire selon la revendication précédente, **caractérisée par le fait que** le ou les blocs hydrophiles représentent an moins 60 % en poids du copolymère linéaire séquence.

3. Composition capillaire selon l'une des revendications précédentes, **caractérisée par le fait que** le copolymère linéaire séquencé est choisi parmi les copolymères diblocs, les copolymères triblocs et les copolymères multiblocs.

4. Composition capillaire selon la revendication 3, **caractérisée par le fait que** le copolymère linéaire séquencé est choisi parmi les copolymères diblocs et les copolymères triblocs comportant un bloc central hydrophile et deux blocs latéraux hydrophobe.

5. Composition capillaire selon l'une quelconque des revendications précédentes, **caractérisée par le fait que** les blocs hydrophiles sont formés de monomères hydrosolubles choisis parmi les monomères hydrosolubles anioniques, les monomères hydrosolubles non-ioniques et les monomères hydrosolubles cationiques ou un mélange de ceux-ci.

6. Composition capillaire selon la revendication 5, **caractérisée par le fait que** les monomères hydrosolubles anioniques sont choisis parmi les acides carboxyliques à insaturation éthylénique, l'acide 2-acrylamido-2-méthylpropanesulfonique, l'acide styrènesulfonique**,** l'acide vinylsulfonique et l'acide vinylphosphonique.

7. Composition capillaire selon la revendication 5, **caractérisée par le fait que** les monomères hydrosolubles non-ioniques sont choisis parmi l'acrylamide, les acrylamides N-alkylés en C₁₋₆ ou N,N-dialkylés en C₁₋₃, l'acrylate de polyéthylèneglycol, le méthacrylate de polyéthylèneglycol, le N-vinylacétamide, le N-méthyl-N-vinylacétamide, le N-vinylformamide, le N-méthyl-N-vinylformamide, les N-vinyllactames comportant un groupe cyclique de 4 à 9 atomes de carbone, l'alcool vinylique, l'oxyde d'éthylène, l'acrylate d'hydroxyéthyle, l'acrylate d'hydroxypropyle, le méthacrylate d'hydroxyéthyle et le méthacrylate d'hydroxypropyle.

8. Composition capillaire selon la revendication 5, **caractérisée par le fait que** les monomères hydrosolubles cationiques sont choisis parmi le chlorure de diméthyldiallylammonium, le chlorure de méthylvinylimidazolium, la 2-vinylpyridine, la 4-vinylpyridine, la 2-méthyl-5-vinylpyridine, la vinylamine, les monomères de formule
H₂C=CR₁-CO-X₂
dans laquelle
R₁ représente un atome d'hydrogène ou un groupe méthyle,
X₂ représente un groupe hydrocarboné linéaire ou ramifié en C₁₋₆ portant au moins une fonction amine primaire, secondaire, tertiaire ou au moins un atome d'azote quaternaire, ou un groupe de formule NHR₂ ou de formule NR₂R₃ où R₂ et R₃ représentent indépendamment l'un de l'autre chacun un groupe hydrocarboné en C_{1-6,} linéaire ou ramifié, portant au moins une fonction amine primaire, secondaire, tertiaire ou au moins un atome d'azote quaternaire.

9. Composition capillaire selon l'une quelconque des revendications précédentes, **caractérisée par le fait que** les blocs hydrophobes sont formés de monomères insolubles dans l'eau choisis parmi les monomères vinylaromatiques, les diènes et les dérivés alkylés des diènes, le chloroprène, les acrylates d'alkyle en C₁₋₁₀, d'aryle en C₈₋₁₀ ou d'aralkyle en C₁₋₁₀, les méthacrylates d'alkyle en C₁₋₁₀, d'aryle en C₆₋₁₀ ou d'aralkyle en C₁₋₁₀, l'acétate de vinyle, les vinyléthers de formule CH₂=CH-O-R et les allyléthers de formule CH₂=CH-CH₂-O-R où R représente un groupe alkyle en C₁₋₆, l'acrylonitrile, le chlorure de vinyle, le chlorure de vinylidène, la caprolactone, l'éthylène, le propylène et les monomères vinyliques fluorés ou à chaîne perfluorée.

10. Composition capillaire selon l'une quelconque des revendications 5 à 8, **caractérisée par le fait que** les blocs hydrophiles contiennent jusqu'à 25 % en moles, de préférence jusqu'à 10 % en moles, et idéalement jusqu'à 5 % en moles, d'un ou de plusieurs monomères insolubles dans l'eau selon la revendication 9.

11. Composition capillaire selon la revendication 7 ou 8, **caractérisée par le fait que** le ou les blocs hydrophobes contiennent jusqu'à 25 % en moles, de préférence jusqu'à 10 % en moles, et idéalement jusqu'à 5 % en moles, d'un ou de plusieurs monomères hydrosolubles selon l'une quelconque des revendications 5 à 8.

12. Composition capillaire selon l'une quelconque des revendications précédentes, **caractérisée par le fait que** le ou les copolymères linéaires séquencés sont présents à raison de 0,1 à 5 % en poids rapporté à la composition capillaire.

13. Composition capillaire selon l'une quelconque des revendications précédentes, **caractérisée par le fait que** les polymères fixants ou conditionneurs sont des polymères cationiques, anioniques, non-ioniques ou amphotères.

14. Composition capillaire selon la revendication 19, **caractérisée par le fait que** les polymères fixants ou conditionneurs cationiques sont choisis parmi les homopolymères ou copolymères d'esters ou d'amides acryliques ou méthacrylique à fonction amine, les polysaccharides à fonctions ammonium quaternaire, les polymères à motifs pipérazinyle et à motifs alkylène ou hydroxyalkylène, les polyaminoamides solubles dans l'eau, les cyclopolymères d'alkyldiallylamine ou de dialkyldiallylammonium, les polymères de diammonium quaternaire, les polymères de polyammonium quaternaire, les polymères quaternaires de vinylpyrrolidone et de vinylimidazole, les polyamines, les polymères de sels de méthacryloyloxyalkyl(C₁₋₄)trialkyl(C₁₋₄)ammonium et les chitosanes.

15. Composition capillaire selon la revendication 13, **caractérisée par le fait que** les polymères fixants ou conditionneurs non-ioniques sont choisis parmi les copolymères de vinylpyrrolidone et de vinylcaprolactame, les homopolymères d'acétate de vinyle, les polyalkyloxazolines, les copolymères d'acétate de vinyle et de maléate d'alkyle, les homopolymères d'acrylate d'alkyle, les homopolymères de méthacrylate d'alkyle, les copolymères d'esters acryliques et méthacryliques, les copolymères d'acrylonitrile et d'un comonomère non-ionique, les polyamides, les polyurèthanes non-ioniques et les polymères siliconés non-ioniques.

16. Composition capillaire selon la revendication 15, **caractérisée par le fait que** les polymères fixants ou conditionneurs anioniques sont choisis parmi les homopolymères ou copolymères d'acide acrylique et méthacrylique ou leurs sels, les copolymères d'acide crotonique, les copolymères d'acides ou d'anhydrides monoinsaturés en C₄₋₈, les polyacrylamides à groupements carboxylate, les homopolymères ou copolymères à groupes sulfoniques, les polyuréthanes anioniques et les polymères siliconés greffés anioniques.

17. Composition capillaire selon la revendication 13, **caracterisée par le fait que** les polymères fixants ou conditionneurs amphotères sont choisis parmi les copolymères à motifs vinyliques acides et à motifs vinyliques basiques, les polyaminoamides réticulés et acylés, les polymères à motifs zwitterioniques, les chitosanes à groupes carboxyle, les copolymères alkyl(C₁₋₅)vinyléther/anhydride maléique modifiés par amidification partielle, les polyuréthanes amphotères et les polymères siliconés greffés amphotères.

18. Composition capillaire selon l'une quelconque des précédentes, **caractérisée par le fait que** le ou les polymères fixant ou conditionneurs sont présents à raison de 0,01 à 20 % en poids, de préférence à raison de 0,1 à 10 % en poids

19. Composition capillaire selon l'une quelconque des revendications précédentes, **caractérisée par le fait qu'**elle contient en outre des additifs cosmétiques et/ou des adjuvants de formulation tels que ies silicones volatiles ou non volatiles, les agents tensioactifs anioniques, cationiques, amphotères ou non-ioniques, les agents nacrants, les agents opacifiants, les pigments et colorants, les huiles, les cires dont les céramides, les filtres UV organiques ou minéraux, les agents anti-radicalaires, les agents plastifiants, les vitamines, les protéines, les agents anti-pelliculaires, les agents d'ajustement et de fixation du pH, les agents anti-oxydants, les agents conservateurs, les précurseurs de colorants capillaires et les agents oxydants.

20. Composition capillaire selon l'une quelconque des revendications précédentes, **caractérisée par le fait qu'**elle se présente sous forme de lotion épaissie, de gel, de crème ou de pâte.

21. Utilisation d'un copolymère linéaire séquencé,qui, introduit dans l'eau à 25 °C, à une concentration en poids égale à 0,1%, permet l'obtention d'une solution ou suspension macroscopiquement homogène et transparente ou translucide, c'est-à-dire ayant une valeur de transmittance de la lumière, à une longueur d'onde égale à 300 nm, à travers un échantillon de 1 cm d'épaisseur, d'au moins 70% comprenant au moins un bloc hydrophile et au moins un bloc hydrophobe, le ou les blocs hydrophiles représentant au moins 30 % en poids du copolymère linéaire séquencé, à l'exclusion des copolymères séquencés d'oxyde d'éthylène et d'oxyde de propylène, des copolymères séquencés à motifs uréthanne et des copolymères séquencés à motifs siloxane, pour l'épaississement ou la gélification de compositions capillaires aqueuses contenant au moins un polymère fixant ou un polymère conditionneur.

## Claims

1. Hair composition containing, in an aqueous cosmetically acceptable medium,
• at least one fixing polymer or conditioning polymer, and
• at least one linear block copolymer which, when introduced into water at 25°C, at a weight concentration equal to 0.1%, produces a macroscopically homogeneous and transparent or translucent solution or suspension, i.e. a solution or suspension having a light transmittance value, at a wavelength equal to 500 nm, through a sample 1 cm thick, of at least 70%, comprising at least one hydrophilic block and at least one hydrophobic block, the hydrophilic block(s) representing at least 30% by weight of the linear block copolymer, with the exclusion of block copolymers of ethylene oxide and of propylene oxide, block copolymers containing urethane units and block copolymers containing siloxane units, in a proportion of from 0.01% to 10% by weight relative to the hair composition.

2. Hair composition according to the preceding claim, **characterized in that** the hydrophilic block(s) represent(s) at least 60% by weight of the linear block copolymer.

3. Hair composition according to either of the preceding claims, **characterized in that** the linear block copolymer is chosen from diblock copolymers, triblock copolymers and multiblock copolymers.

4. Hair composition according to Claim 3, **characterized in that** the linear block copolymer is chosen from diblock copolymers and triblock copolymers comprising a hydrophilic central block and two hydrophobic side blocks.

5. Hair composition according to any one of the preceding claims, **characterized in that** the hydrophilic blocks are formed from water-soluble monomers chosen from anionic water-soluble monomers, nonionic water-soluble monomers and cationic water-soluble monomers, or a mixture thereof.

6. Hair composition according to Claim 5, **characterized in that** the anionic water-soluble monomers are chosen from ethylenically unsaturated carboxylic acids, 2-acrylamido-2-methylpropanesulphonic acid, styrenesulphonic acid, vinylsulphonic acid and vinylphosphonic acid.

7. Hair composition according to Claim 5, **characterized in that** the nonionic water-soluble monomers are chosen from acrylamide, N-C₁₋₆ alkyl or N,N-di-C₁-₃ alkyl acrylamides, polyethylene glycol acrylate, polyethylene glycol methacrylate, N-vinylacetamide, N-methyl-N-vinylacetamide, N-vinylformamide, N-methyl-N-vinylformamide, N-vinyllactams comprising a cyclic group of 4 to 9 carbon atoms, vinyl alcohol, ethylene oxide, hydroxyethyl acrylate, hydroxypropyl acrylate, hydroxyethyl methacrylate and hydroxypropyl methacrylate.

8. Hair composition according to Claim 5, **characterized in that** the cationic water-soluble monomers are chosen from dimethyldiallylammonium chloride, methylvinylimidazolium chloride, 2-vinylpyridine, 4-vinylpyridine, 2-methyl-5-vinylpyridine, vinylamine and the monomers of formula
H₂C=CR₁-CO-X₂
in which
R₁ represents a hydrogen atom or a methyl group,
X₂ represents a linear or branched C₁₋₆ hydrocarbon-based group bearing at least one primary, secondary or tertiary amine function or at least one quaternary nitrogen atom, or a group of formula NHR₂ or of formula NR₂R₃ in which R₂ and R₃ represent, independently of each other, a linear or branched C₁-₆ hydrocarbon-based group bearing at least one primary, secondary or tertiary amine function or at least one quaternary nitrogen atom.

9. Hair composition according to any one of the preceding claims, **characterized in that** the hydrophobic blocks are formed from water-insoluble monomers chosen from vinylaromatic monomers, dienes and alkyl derivatives of dienes, chloroprene, C₁₋₁₀ alkyl, C₆₋₁₀ aryl or C₁₋₁₀ aralkyl acrylates, C₁₋₁₀ alkyl, C₆₋₁₀ aryl or C₁₋₁₀ aralkyl methacrylates, vinyl acetate, vinylethers of formula CH₂=CH-O-R and allylethers of formula CH₂=CH-CH₂-O-R in which R represents a C₁₋₆ alkyl group, acrylonitrile, vinyl chloride, vinylidene chloride, caprolactone, ethylene, propylene and vinyl monomers that are fluorinated or that contain a perfluoro chain.

10. Hair composition according to any one of Claims 5 to 8, **characterized in that** the hydrophilic blocks contain up to 25 mol%, preferably up to 10 mol% and ideally up to 5 mol%, of one or more water-insoluble monomers according to Claim 9.

11. Hair composition according to Claim 7 or 8, **characterized in that** the hydrophobic block or blocks contain up to 25 mol%, preferably up to 10 mol% and ideally up to 5 mol%, of one or more water-soluble monomers according to any one of Claims 5 to 8.

12. Hair composition according to any one of the preceding claims, **characterized in that** the linear block copolymer(s) is (are) present in a proportion of from 0.1% to 5% by weight relative to the hair composition.

13. Hair composition according to any one of the preceding claims, **characterized in that** the fixing polymers or conditioning polymers are cationic, anionic, nonionic or amphoteric polymers.

14. Hair composition according to Claim 13, **characterized in that** the cationic fixing polymers or conditioning polymers are chosen from homopolymers or copolymers of acrylic or methacrylic esters or amides containing an amine function, polysaccharides containing quaternary ammonium functions, polymers containing piperazinyl units and alkylene or hydroxyalkylene units, water-soluble polyamino amides, alkyldiallylamine or dialkyldiallylammonium cyclopolymers, diquaternary ammonium polymers, polyquaternary ammonium polymers, quaternary polymers of vinylpyrrolidone and of vinylimidazole, polyamines, polymers of methacryloyloxy(C₁₋₄)alkyltri(C₁₋₄)alkylammonium salts, and chitosans.

15. Hair composition according to Claim 13, **characterized in that** the nonionic fixing polymers or conditioning polymers are chosen from copolymers of vinylpyrrolidone and of vinylcaprolactam, vinyl acetate homopolymers, polyalkyloxazolines, copolymers of vinyl acetate and of alkyl maleate, alkyl acrylate homopolymers, alkyl methacrylate homopolymers, copolymers of acrylic and methacrylic esters, copolymers of acrylonitrile and of a nonionic comonomer, polyamides, nonionic polyurethanes and nonionic silicone polymers.

16. Hair composition according to Claim 13, **characterized in that** the anionic fixing polymers or conditioning polymers are chosen from homopolymers or copolymers of acrylic and methacrylic acid or salts thereof, crotonic acid copolymers, C₄₋₈ monounsaturated acid or anhydride copolymers, polyacrylamides containing carboxylate groups, homopolymers or copolymers containing sulphonic groups, anionic polyurethanes and anionic grafted silicone polymers.

17. Hair composition according to Claim 13, **characterized in that** the amphoteric fixing polymers or conditioning polymers are chosen from copolymers containing acidic vinyl units and basic vinyl units, crosslinked and acylated polyamino amides, polymers containing zwitterionic units, chitosans containing carboxyl groups, (C₁₋₅)alkyl vinyl ether/maleic anhydride copolymers modified by partial amidation, amphoteric polyurethanes and amphoteric grafted silicone polymers.

18. Hair composition according to any one of the preceding claims, **characterized in that** the fixing or conditioning polymer or polymers that are beneficial for the hair are present in a proportion of from 0.01% to 20% by weight and preferably in a proportion of from 0.1% to 10% by weight.

19. Hair composition according to any one of the preceding claims, **characterized in that** it also contains cosmetic additives and/or formulation adjuvants such as volatile or non-volatile silicones, anionic, cationic, amphoteric or nonionic surfactants, nacreous agents, opacifiers, pigments and colorants, oils, waxes including ceramides, organic or mineral UV-screening agents, free-radical scavengers, plasticizers, vitamins, proteins, antidandruff agents, agents for adjusting and fixing the pH, antioxidants, preserving agents, hair dye precursors and oxidizing agents.

20. Hair composition according to any one of the preceding claims, **characterized in that** it is in the form of a thickened lotion, a gel, a cream or a paste.

21. Use of a linear block copolymer which, when introduced into water at 25°C, at a weight concentration equal to 0.1%, produces a macroscopically homogeneous and transparent or translucent solution or suspension, i.e. a solution or suspension having a light transmittance value, at a wavelength equal to 500 nm, through a sample 1 cm thick, of at least 70%, comprising at least one hydrophilic block and at least one hydrophobic block, the hydrophilic block(s) representing at least 30% by weight of the linear block copolymer, with the exclusion of block copolymers of ethylene oxide and of propylene oxide, block copolymers containing urethane units and block copolymers containing siloxane units, to thicken or gel aqueous hair compositions containing at least one fixing polymer or conditioning polymer.

## Patentansprüche

1. Zusammensetzung für die Haarbehandlung, die in einem kosmetisch akzeptablen, wässrigen Medium enthält:
• mindestens ein festigendes Polymer oder ein konditionierendes Polymer, und
• mindestens ein lineares Sequenzcopolymer, mit dem, wenn es bei 25 °C in einer Konzentration von 0,1 Gew.-% in Wasser gegeben wird, eine makroskopisch homogene und transparente oder durchscheinende Lösung oder Suspension erhalten wird, d. h. mit einem Wert der Lichtdurchlässigkeit bei einer Wellenlänge von 500 nm durch eine Probe von 1 cm Dicke von mindestens 70 %, das mindestens einen hydrophilen Block und mindestens einen hydrophoben Block aufweist, wobei der oder die hydrophilen Blöcke mindestens 30 Gew.-% des linearen Sequenzcopolymers ausmachen, mit Ausnahme von Sequenzcopolymeren von Ethylenoxid und Propylenoxid, Sequenzcopolymeren mit Urethaneinheiten und Sequenzcopolymeren mit Siloxaneinheiten, und das in einer Menge von 0,01 bis 10 Gew.-% in die Zusammensetzung für die Haarbehandlung eingearbeitet wird.

2. Zusammensetzung für die Haarbehandlung nach dem vorhergehenden Anspruch, **dadurch gekennzeichnet, dass** der oder die hydrophilen Blöcke mindestens 60 Gew.-% des linearen Sequenzcopolymers ausmachen.

3. Zusammensetzung für die Haarbehandlung nach einem der vorhergehenden Ansprüche, **dadurch gekennzeichnet, dass** das lineare Sequenzcopolymer unter den Zweiblock-Copolymeren, Dreiblock-Copolymeren und Multiblock-Copolymeren ausgewählt ist.

4. Zusammensetzung für die Haarbehandlung nach Anspruch 3, **dadurch gekennzeichnet, dass** das lineare Sequenzcopolymer unter den Zweiblock-Copolymeren und den Dreiblock-Copolymeren ausgewählt ist, die einen zentralen hydrophilen Block und zwei hydrophobe Seitenblöcke aufweisen.

5. Zusammensetzung für die Haarbehandlung nach einem der vorhergehenden Ansprüche, **dadurch gekennzeichnet, dass** die hydrophilen Blöcke aus wasserlöslichen Monomeren gebildet sind, die unter den wasserlöslichen anionischen Monomeren, wasserlöslichen nichtionischen Monomeren und wasserlöslichen kationischen Monomeren oder einem Gemisch aus diesen Monomeren ausgewählt sind.

6. Zusammensetzung für die Haarbehandlung nach Anspruch 5, **dadurch gekennzeichnet, dass** die anionischen wasserlöslichen Monomere unter den Carbonsäuren mit ethylenisch ungesättigter Bindung, 2-Acrylamido-2-methylpropansulfonsäure, Styrolsulfonsäure, Vinylsulfonsäure und Vinylphosphonsäure ausgewählt sind.

7. Zusammensetzung für die Haarbehandlung nach Anspruch 5, **dadurch gekennzeichnet, dass** die nichtionischen wasserlöslichen Monomere unter Acrylamid, N-Alkyl(C₁₋₆)acrylamiden oder N,N-Dialkyl(C₁₋₃)acrylamiden, Polyethylenglycolacrylat, Polyethylenglycolmethacrylat, N-Vinylacetamid, N-Methyl-N-vinylacetamid, N-Vinylformamid, N-Methyl-N-vinylformamid, N-Vinyllactamen, die eine cyclische Gruppe mit 4 bis 9 Kohlenstoffatomen aufweisen, Vinylalkohol, Ethylenoxid, Hydroxyethylacrylat, Hydroxypropylacrylat, Hydroxyethylmethacrylat und Hydroxypropylmethacrylat ausgewählt sind.

8. Zusammensetzung für die Haarbehandlung nach Anspruch 5, **dadurch gekennzeichnet, dass** die kationischen wasserlöslichen Monomere unter Dimethyldiallylammoniumchlorid, Methylvinylimidazoliumchlorid, 2-Vinylpyridin, 4-Vinylpyridin, 2-Methyl-5-vinylpyridin, Vinylamin, Monomeren der Formel
H₂C=CRi-CO-X₂
worin bedeuten:
R₁ ein Wasserstoffatom oder die Methylgruppe,
X₂ eine geradkettige oder verzweigte Kohlenwasserstoffgruppe mit 1 bis 6 Kohlenstoffatomen, die mindestens eine primäre, sekundäre, tertiäre Aminofunktion oder mindestens ein quartäres Stickstoffatom aufweist, eine Gruppe der Formel NHR₂ oder der Formel NR₂R₃, wobei R₂ und R₃ unabhängig voneinander eine geradkettige oder verzweigte C₁₋₆-Kohlenwasserstoffgruppe bedeuten, die mindestens eine primäre, sekundäre, tertiäre Aminofunktion oder mindestens ein quartäres Stickstoffatom aufweist.

9. Zusammensetzung für die Haarbehandlung nach einem der vorhergehenden Ansprüche, **dadurch gekennzeichnet, dass** die hydrophoben Blöcke aus in Wasser unlöslichen Monomeren gebildet sind, die unter den vinylaromatischen Monomeren, Dienen und alkylierten Dienderivaten, Chloropren, C₁₋₁₀-Alkylacrylaten, C₆₋₁₀-Arylacrylaten oder C₁₋₁₀-Aralkylacrylaten, C₁₋₁₀-Alkylmethacrylaten, C₆₋₁₀-Arylmethacrylaten oder C₁₋₁₀-Aralkylmethacrylaten, Vinylacetat, Vinylethern der Formel CH₂=CH-O-R und Allylethern der Formel CH₂=CH-CH₂-O-R, worin R eine C₁₋₆-Alkylgruppe bedeutet, Acrylnitril, Vinylchlorid, Vinylidenchlorid, Caprolacton, Ethylen, Propylen und fluorierten Vinylmonomeren oder Vinylmonomeren mit perfluorierter Kette ausgewählt sind.

10. Zusammensetzung für die Haarbehandlung nach einem der Ansprüche 5 bis 8, **dadurch gekennzeichnet, dass** die hydrophilen Blöcke bis zu 25 Mol-%, vorzugsweise bis zu 10 Mol-% und idealerweise bis zu 5 Mol-% eines oder mehrerer in Wasser unlöslicher Monomere nach Anspruch 9 enthalten.

11. Zusammensetzung für die Haarbehandlung nach Anspruch 7 oder 8, **dadurch gekennzeichnet, dass** der oder die hydrophoben Blöcke bis zu 25 Mol-%, vorzugsweise bis zu 10 Mol-% und idealerweise bis zu 5 Mol-% eines oder mehrerer wasserlöslicher Monomere nach einem der Ansprüche 5 bis 8 enthalten.

12. Zusammensetzung für die Haarbehandlung nach einem der vorhergehenden Ansprüche, **dadurch gekennzeichnet, dass** das oder die linearen Sequenzcopolymere in einer Menge von 0,1 bis 5 Gew.-%, bezogen auf das Gesamtgewicht die Zusammensetzung für die Haarbehandlung, enthalten sind.

13. Zusammensetzung für die Haarbehandlung nach einem der vorhergehenden Ansprüche, **dadurch gekennzeichnet, dass** die festigenden Polymere oder die konditionierenden Polymere kationische, anionische, nichtionische oder amphotere Polymere sind.

14. Zusammensetzung für die Haarbehandlung nach Anspruch 13, **dadurch gekennzeichnet, dass** die festigenden oder konditionierenden kationischen Polymere unter den Homopolymeren oder Copolymeren von Acrylsäureestern, Methacrylsäureestern, Acrylamiden oder Methacrylamiden mit Aminofunktion, Polysacchariden mit quartärer Ammoniumfunktion, Polymeren mit Piperazinyleinheiten und Alkylen- oder Hydroxyalkyleneinheiten, in Wasser löslichen Polyaminoamiden, Alkyldiallylamin-Cyclopolymeren oder Dialkyldiallylammönium-Cyclopolymeren, quartärem Diammoniumpolymeren, Polymeren mit quartärem Polyammonium, quartären Polymeren von Vinylpyrrolidon und Vinylimidazol, Polyaminen, Polymeren von Methacryloyloxyalkyl(C₁₋₄)trialkyl(C₁₋₄)ammoniumsalzen und Chitosanen ausgewählt sind.

15. Zusammensetzung für die Haarbehandlung nach Anspruch 13, **dadurch gekennzeichnet, dass** die festigenden oder konditionierenden nichtionischen Polymere unter den Copolymeren von Vinylpyrrolidon und Vinylcaprolactam, Homopolymeren von Vinylacetat, Polyalkyloxazolinen, Copolymeren von Vinylacetat und Alkylmaleat, Homopolymeren von Alkylacrylat, Homopolymeren von Alkylmethacrylat, Copolymeren von Acrylsäureestern und Methacrylsäureestern, Copolymeren von Acrylnitril und einem nichtionischen Comonomer, Polyamiden, nichtionischen Polyurethanen und nichtionischen siliconierten Polymeren ausgewählt sind.

16. Zusammensetzung für die Haarbehandlung nach Anspruch 13, **dadurch gekennzeichnet, dass** die festigenden oder konditionierenden anionischen Polymere unter den Homopolymeren oder Copolymeren von Acrylsäure und Methacrylsäure oder deren Salzen, Copolymeren von Crotonsäure, Copolymeren von einfach ungesättigten Säuren oder Anhydriden mit 4 bis 8 Kohlenstoffatomen, Polyacrylamiden mit Carboxylatgruppen, Homopolymeren oder Copolymeren mit Sulfonsäuregruppen, anionischen Polyurethanen und anionischen gepfropften Siliconpolymeren ausgewählt sind.

17. Zusammensetzung für die Haarbehandlung nach Anspruch 13, **dadurch gekennzeichnet, dass** die festigenden oder konditionierenden amphoteren Polymere unter den Copolymeren mit sauren Vinyleinheiten und basischen Vinyleinheiten, vernetzten und acylierten Polyaminoamiden, Polymeren mit zwitterionischen Einheiten, Chitosanen mit Carboxygruppen, Alkyl(C₁₋₅)vinylether/Maleinsäureanhydrid-Copolymeren, die durch teilweise Amidierung modifiziert sind, amphoteren Polyurethanen und amphoteren gepfropften Siliconpolymeren ausgewählt sind.

18. Zusammensetzung für die Haarbehandlung nach einem der vorhergehenden Ansprüche, **dadurch gekennzeichnet, dass** das oder die festigenden oder konditionierenden Polymere in einer Menge von 0,01 bis 20 Gew.-% und vorzugsweise 0,1 bis 10 Gew.-% enthalten sind.

19. Zusammensetzung für die Haarbehandlung nach einem der vorhergehenden Ansprüche, **dadurch gekennzeichnet, dass** sie ferner kosmetische Zusatzstoffe und/oder Formulierungshilfsstoffe enthält, wie flüchtige oder nicht flüchtige Silicone, anionische, kationische, amphotere oder nichtionische grenzflächenaktive Stoffe, Perlglanzstoffe, Trübungsmittel, Pigmente und Farbstoffe, Öle, Wachse, darunter Ceramide, organische oder anorganische UV-Filter, Radikalfänger für freie Radikale, Weichmacher, Vitamine, Proteine, Antischuppenmittel, Stoffe zum Einstellen und Halten des pH-Werts, Antioxidantien, Konservierungsmittel, Farbstoffvorprodukte von Haarfarbstoffen und Oxidationsmitteln.

20. Zusammensetzung für die Haarbehandlung nach einem der vorhergehenden Ansprüche, **dadurch gekennzeichnet, dass** sie in Form einer dickflüssigen Lotion, als Gel, als Creme oder als Paste vorliegt.

21. Verwendung eines linearen Sequenzcopolymers, mit dem, wenn es bei einer Konzentration von 0,1 Gew.-% in Wasser von 25 °C eingebracht wird, eine makroskopisch homogene und transparente oder durchscheinende Lösung oder Suspension erhalten werden kann, d. h. mit einem Wert der Lichtdurchlässigkeit bei einer Wellenlänge von 500 nm durch eine Probe von 1 cm Dicke von mindestens 70 %, und das mindestens einen hydrophilen Block und mindestens einen hydrophoben Block aufweist, wobei der oder die hydrophilen Blöcke mindestens 30 Gew.-% des linearen Sequenzcopolymers ausmachen, wobei Sequenzcopolymere von Ethylenoxid und Propylenoxid, Sequenzcopolymere mit Urethaneinheiten und Sequenzcopolymere mit Siloxaneinheiten ausgenommen sind, zum Verdicken oder Gelieren von wässrigen Zusammensetzungen für die Haarbehandlung, die mindestens ein festigendes Polymer oder ein konditionierendes Polymer enthalten.
